# EUROPEAN PATENT SPECIFICATION

(11) **EP 1 853 284 B1**
(45) Date of publication and mention of the grant of the patent: **09.04.2014**
(21) Application number: 06705161.5
(22) Date of filing: 10.02.2006
(51) Int. Cl.: A61K 35/74, A23C 21/02, A23C 9/123, A61P 1/00, A61P 29/00

(54) **USE OF LACTOBACILLUS KEFIRANOFACIENS AS A PROBIOTIC AND A SYNBIOTIC**
VERWENDUNG VON LACTOBACILLUS KEFIRANOFACIENS ALS PROBIOTIKUM UND SYNBIOTIKUM
UTILISATION DE LACTOBACILLUS KEFIRANOFACIENS COMME PROBIOTIQUE ET SYNBIOTIQUE

(30) Priority: 11.02.2005 US 651657 P
(43) Date of publication of application: 14.11.2007
(73) Proprietor: Biolactis Inc., Nassau (BS)
(72) Inventor: LEMIEUX, Pierre, Sainte-Thérèse, Québec J7E 4Z2 (CA); PRECOURT, Louis-Philippe, Quebec J7H 1K9 (CA); SIMARD, Eric, Laval, Quebec J7N3J8 (CA)
(74) Representative: Brown, David Leslie
(86) International application number: PCT/CA2006/000206
(87) International publication number: WO 2006/084381

(56) References cited:
- EP-A2- 1 243 274
- WO-A1-01/88095
- WO-A2-03/053158
- US-B2- 6 733 801
- LEMIEUX P ET AL: "Socioeconomic and health benefits of lactoceuticals", EXPERT REVIEW OF PHARMACOECONOMICS AND OUTCOMES RESEARCH 200404 GB, vol. 4, no. 2, April 2004 (2004-04), pages 199-206, XP009156514, ISSN: 1473-7167
- NURUZOVA Z A ET AL: "Disbacteriosis in gastrointestinal diseases and their biocorrection", UZBEKISTON TIBBIET ZHURNALI, no. 3, May 2000 (2000-05), pages 25-28, XP009156513,
- MAEDA H. ET AL.: 'Structural Characterization and Biological Activities of an Exopolysaccharide Kefiran Produced by Lactobacillus kefiranofaciens WT-2B' J. AGRIC. FOOD CHEM. vol. 52, no. 17, August 2004, pages 5533 - 5538, XP008120693
- MAEDA H. ET AL.: 'Effects of an exopolysaccharide (kefiran) on lipids, blood pressure, blood glucose, and constipation' BIOFACTORS vol. 22, 2004, pages 197 - 200, XP008110634
- SANTOS A. ET AL.: 'The Antimicrobial Properties of Different Strains of Lactobacillus spp. Isolated from Kefir' SYSTEM APPL. MICROBIOL. vol. 26, September 2003, pages 434 - 437, XP004957460
- BAHARAV E. ET AL.: 'Lactobacillus GG Bacteria Ameliorate Arthritis in Lewis Rats' J. NUTR. vol. 134, August 2004, pages 1964 - 1969, XP008111136

## Description

### BACKGROUND OF THE INVENTION

### (a) Field of the Invention

This invention relates to the use of *Lactobacillus kefiranofaciens* as a probiotic having effects on intestinal health obesity-associated problem such as control of blood lipid levels.

### (b) Description of Prior Art

The traditional definition of a probiotic microorganism requires that the bacteria (and its components) be non-toxic, survive through gastric/intestinal environments, adhere/persist in the gastro-intestinal tract, exist as part of the normal human microflora and exert health benefits. However, recent evidence has shown that some probiotic effects can be obtained from lactobacilli of non-human origin and that probiotic effects can be obtained with *lactobacillus* that were heat or radiation inactivated, and in some case from bacterial lysates (U.S. Pat. No 4,347,240). The health benefits observed from probiotic lactobacilli are often strain specific and can vary greatly. Some of the effects described for probiotic lactobacilli include: modulation of intestinal microflora, competition with and elimination of pathogenic microorganisms, modulation of immune function, control of allergies, promotion of gastrointestinal health, regulation of blood lipid levels, control of diabetes (regulation of glucose and insulin in blood), protection against colon cancer and control of body weight.

Kefir has been used to ferment milk for centuries. Kefir grains are composed of Gram-positive hetero- and homofermentive lactic acid bacteria. Gram-negative acetic acid bacteria, and lactose fermenting and non-fermenting yeasts, held together by kefiran, a biopolymer of the exopolysaccharide family secreted by *Lactobacillus kefiranofaciens* sub-specie (subsp.) *kefiranofaciens* bacteria. Lactobacilli of the *L*. *kefiranofaciens* species are homofermentive lactobacilli and represent the major bacterial population of kefir grains. While originally classified as 2 independent species, *Lactobacillus kefiranofaciens* and *Lactobacillus kefirgranum* were recently re-classified as sub-species of the *L*. *kefiranofaciens* species based on their identical 16S RNA sequences (Vancanneyt et al., Int J Syst Evol Microbiol. 54(Pt 2):551-556, 2004). The classification to the two subspecies is done on the basis of morphology on agar plates and in liquid culture, on acid production from different sugars, and on protein profiling from PAGE. Strains from the subspecies *kefiranofaciens* are usually high producers of kefiran, which is essential in the composition and formation of kefir grains. Kefiran production from *kefiranofaciens* strains is recognizable from the colony morphology (showing glossy or slimy appearance) on agar plates, while strains from the subspecies *kefirgranum* (showing dry and compact colonies) do not produce significant levels of kefiran. However, kefiran production from *Lactobacillus kefiranofaciens* subsp. *kefiranofaciens* has been shown to be very sensitive to subculturing. *Kefirgranum* strains can produce acid from threalose, while *kefiranofaciens* strains cannot. In addition, strains from the *kefirgranum* sub-species are flocculent and sediment in liquid cultures.

The fact that no toxicity has been associated with kefir over the years is a strong argument to the safety and non-toxicity of lactobacillus strains isolated from it. The species *Lactobacillus kefiranofaciens* has been classified in the *L. acidophilus* group phylogenically close to *L*. *crispatus* and *L*. *acidophilus* species, which contain several well described probiotic strains.

Santos et al. (System. Appl. Microbiol., 26:434-437, 2003) describe four strains of *Lactobacillus kefiranofaciens* which show resistance to acid and bile, and different adhesion and antimicrobial properties.

U.S. Patent No 4,347,240 describes the isolation of a novel strain of *lactobacillus* KPB-176 of undefined strain classification (*Lactobacillus kefiranofaciens* subsp. *kefiranofaciens*) from kefir grains, which produces large quantities of kefiran, does not possess strict selectivity for specific media and involves no reduction in the productivity of polysaccharides even during subculture.

WO030 53158 discloses malleable protein matrix comprising a micro organism that can be chosen from Lactobacillus kefiranofaciens.

### SUMMARY OF THE INVENTION

In accordance with the present invention there is provided a probiotic composition comprising an effective amount of *Lactobacillus kefiranofaciens* in association with a suitable carrier as defined in the claims.

The *Lactobacillus kefiranofaciens* is a strain selected from the group consisting of R2C2 (IDAC accession number 041202-3), INIX (IDAC accession number 041202-4), K2 (IDAC accession number 041202-1); ES1 (IDAC accession number 041202-2).

The probiotic effect is protection against Intestinal inflammation. The composition may be suitably formulated for oral, rectal or vaginal administration.

Still in accordance with the present disclosure, there is also provided a method for providing positive modulation of the intestinal microflora in a subject comprising the step of administering to said subject an effective amount of such *Lactobacillus kefiranofaciens.*

Also in accordance with the present invention, there is provided a composition for use in a method for protecting a subject against intestinal inflammation comprising the step of administering to said subject an effective amount of *Lactobacillus kefiranofaciens.* Such intestinal inflammation can be caused for example by an inflammatory bowel disease (IBD), Crohn's disease (CD), ulcerative colitis (UC) or by an irritable bowel syndrome (IBS).

Further in accordance with the present disclosure, there is provided a method for protecting a subject against allergies and/or autoimmune diseases comprising the step of administering to said subject an effective amount of such *Lactobacillus kefiranofaciens.*

Also in accordance with the present disclosure, there is also provided a method for protecting a subject against diarrhea comprising the step of administering to said subject an effective amount of such *Lactobacillus kefiranofaciens*.

According to the present disclosure, there is still provided a method for protecting a subject against diabetes comprising the step of administering to said subject an effective amount of such *Lactobacillus kefiranofaciens.*

There is also provided in accordance to the present disclosure a method for protecting a subject against hyperlipidemia comprising the step of administering to said subject an effective amount of such *Lactobacillus* kefrranofaciens.

Still in accordance with the present disclosure, there is also provided a method for protecting a subject against colon cancer comprising the step of administering to said subject an effective amount of such *Lactobacillus kefiranofaciens.*

In one embodiment, the *Lactobacillus kefiranofaciens* is administered in a form selected from the group consisting of a live bacterial population, a lyophilized bacterial population, as a fermented dairy product and as a non-viable bacterial sample, such as a heat-killed bacteria, an irradiated bacteria or a lysed bacteria.

One embodiment of the present disclosure further provides for the use of such *Lactobacillus kefiranofaciens* as a probiotic compound having an anti-inflammatory effect.

One embodiment of the disclosure provides for the use of such *Lactobacillus kefiranofaciens* as a probiotic compound for treating psoriasis.

In accordance with the present invention, there is also provided the use of *Lactobacillus kefiranofaciens* in association with an anti-inflammatory compound, wherein the inflammatory compound is 5-ASA or a corticosteroid.

The present disclosure further provides for the use of *Lactobacillus kefiranofaciens* as a probiotic compound for the manufacture of a medicament for treating psoriasis.

In one embodiment of the disclosure, the *Lactobacillus kefiranofaciens* is used to ferment whey, wherein said whey is cheese whey.

In accordance with the present disclosure, there is also provided the use of a product obtained from the fermentation of whey by *Lactobacillus kefiranofaciens* for the treatment of a cardiovascular disease.

In accordance with the present disclosure, there is also provided the use of a product obtained from the fermentation of whey by *Lactobacillus kefiranofaciens* for the treatment of hypertension.

The present disclosure further provides for the use of a product obtained from the fermentation of whey by *Lactobacillus kefiranofaciens* for the treatment of weight disorder.

In accordance with the present disclosure, there is also provided the use of a product obtained from the fermentation of whey by *Lactobacillus kefiranofaciens* for the treatment of hyperlipidemia

The present disclosure further provides for the use of a product obtained from the fermentation of whey by *Lactobacillus kefiranofaciens* for the treatment of triglyceride disorder.

The present disclosure further provides for the use of such *Lactobacillus kefiranofaciens* as a probiotic compound. The probiotic compound may be used for treating metabolic syndrome which is associated with 5 problems which are hypertension, low HDL, fat in belly, insulin resistance and high level of triglyceride. Thus, the probiotic compound of the present invention can be used for treating and/or preventing the problems associated with metabolic syndrome.

The present disclosure further provides for the use of such *Lactobacillus kefiranofaciens* as a probiotic compound for controlling weight management.

### DETAILED DESCRIPTION OF THE INVENTION

In accordance with the present invention, there is provided probiotic lactobacilli with various applications in foodstuffs and in medicine. The invention relates to a probiotic species *Lactobacillus kefiranofaciens* (including *Lactobacillus kefiranofaciens* subsp. *kefiranofaciens* and *Lactobacillus kefiranofaciens* subsp, *Kefirgranum),* defined by the 4 strains described below, which have shown significant probiotic potential following *per os* treatment in animal models. Among these probiotic effects it was observed intestinal adherence, positive modulation of the intestinal microflora, immunomodulation, allergies, diarrhea, weight management, hypertension and hyperlipidemia. The different strains isolated from this species have shown both common and strain specific beneficial health effects. They can be administered orally either as live or lyophilized bacterial population, as a fermented dairy product (milk or whey based) or as non-viable bacterial samples (heat-killed, irradiated or lysed bacteria).

The present invention will be more readily understood by referring to the following examples which are given to illustrate the invention rather than to limit its scope.

Some of the examples have been included for reference purpose only.

### Example 1

### Identification of five probiotic strains of lactobacillus from kefir grains

Three of the strains described in the present application, R2C2 (IDAC accession number 041202-3), K2 (IDAC accession number 041202-1) and ES1 (IDAC accession number 041202-2) were isolated from kefir grain previously adapted for growth in whey.

One strain, INIX (IDAC accession number 041202-4) was isolated from a kefiran-overproducing colony on agar plates of the ATCC *kefiranofaciens* reference strain (ATCC accession number 43761).

BioSp was a *Lactobacillus kefiranofaciens* subsp, *kefirgranum* strain strongly flocculent in liquid cultures from Technologie Biolactis inc.

Based on their 16S RNA gene sequences, the strains described in the present application have been assigned to the species *Lactobacillus kefiranofaciens.* Morphological properties in liquid culture and on agar plates and carbohydrate fermentation profile have also permitted a more specific assignment of the strains to either *Lactobacillus kefiranofaciens* subsp. *kefiranofaciens* (R2C2, ES1 and INIX) and to *Lactobacillus kefiranofaciens* subsp. *kefirgranum* (K2 and BioSp).

### Example 2

### In vitro characterization of the lactobacillus strains

R2C2: generally short bacilli, gram positive, single or in chains of 2 to 4, not producing or producing few exopolysaccharides. The length and the width of the bacilli vary much according to the culture medium used and the medium of conservation. On RCW plates, it forms dry and smooth, crystalline looking colonies, white with beige center, convex, with embossed top. This strain has a good growth in poor mediums (for example in whey not supplemented) and produces agglomerates of small sizes in liquid culture (approximately 10 bacteria and less).

INIX: elongated, generally longer bacilli than R2C2, gram positive, single or in chains of 2 to 4 bacilli. On RCW plate, it forms slimy or sticky looking colonies, which fuse to neighboring colonies. Kefiran production rates in liquid RCW cultures are high and stable upon subculturing. This strain has a weak growth in poor mediums; typical to *Lactobacillus kefiranofaciens* subsp. *kefiranofaciens* strains and produce agglomerates of small sizes in liquid culture (approximately 10 to 50 bacteria).

ES1: Similar to R2C2 strain, but presents a worse growth in poor mediums.

K2: Bacilli of average length slightly wither than R2C2, single or in chains of 2 to 4 bacilli. Colonies similar to R2C2. Does not produce or few exopolysaccharides. Growth in poor medium, but reached the stationary phase at level lower than the other strains for all the culture media tested (RCW: 5 to 7 X 10⁸ bacteria compared to 1,5 to 3 X 10⁹ for R2C2 and ES1). Presents agglomerates of average size in liquid culture (approximately 50 to 100 bacteria).

BioSP: Bacilli of average length, with irregular cells surface, wither than all other strains, single or in chains of 2 to 4 bacilli, not producing or few exopolysaccharides. Gram positive, formation of large size aggregates of lactobacilli in liquid culture also containing precipitated proteins (more than 100 bacteria). On RCW plates, it forms white colonies, convex, with the more uniform top, but presenting fine white points on the surface. Growth in poor mediums, but strongly reduced in the presence of strong calcium concentration (1 % CaCl2 p/v).

### Fermentation profiles

Table 1 provides data about the fermentation profiles of the strains described above.

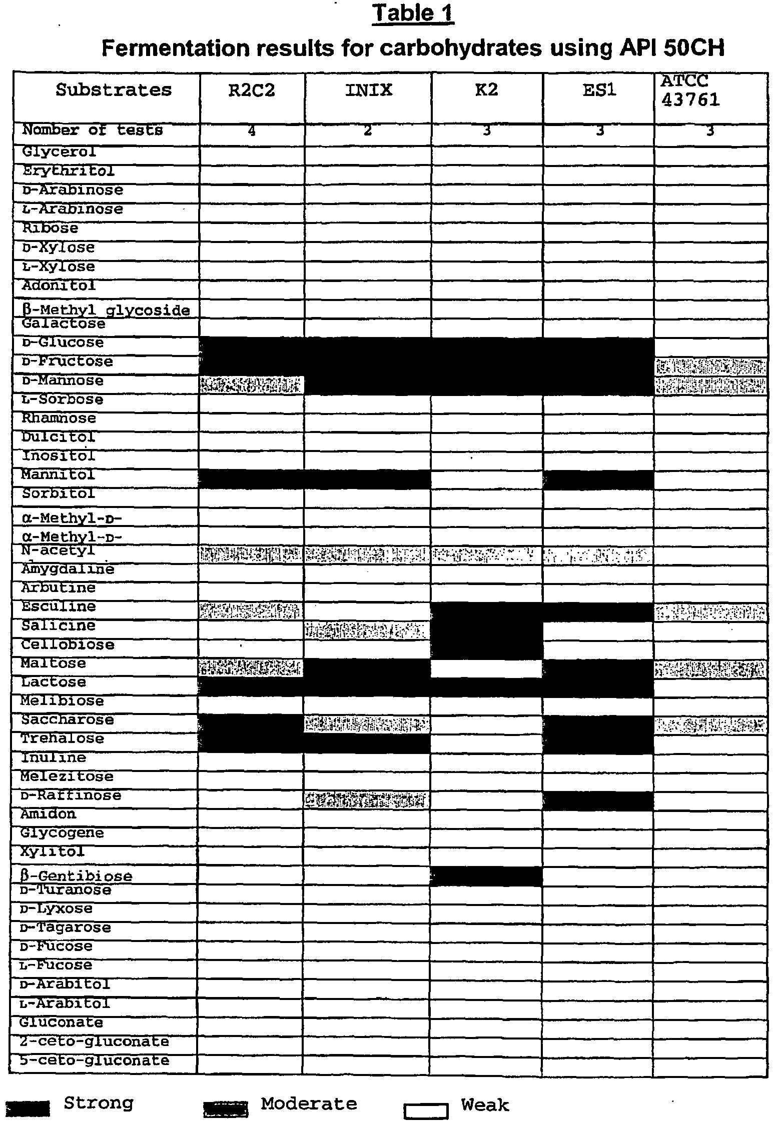

### Metabolite fermentation profiles

Tables 2 to 4 provide data about metabolite fermentation (scale of 1 to 5 defined as 1 being weak to 5 being strong, + = 5 and - means no reaction.

**Table 2**

| **Metabolite fermentation for the strain R2C2** | | | | | | | | | | | | | | | | |
|---|---|---|---|---|---|---|---|---|---|---|---|---|---|---|---|---|
| | JM 3 | | | | JM 19 | | | | 2 JM7 | | | | JJFT | | | |
| Incubation time (h) | 24 | 48 | 72 | 96 | 24 | 48 | 72 | 96 | 24 | 48 | 72 | 96 | 24 | 48 | 72 | 96 |
| | | | | | | | | | | | | | | | | |
| Glycerol | | | | | | | | | | | | | | | | |
| Erythritol | | | | | | | | | | | | | | | | |
| D-Arabinose | | | | | | | | | | | | | | | | |
| L-Arabinose | | | | | | | | | | | | | | | | |
| Ribose | | | | | | | | | | | | | | | | |
| D-Xylose | | | | | | | | | | | | | | | | |
| L-Xylose | | | | | | | | | | | | | | | | |
| Adonitol | | | | | | | | | | | | | | | | |
| β-Methyl glycoside | | | | | | | | | | | | | | | | |
| Galactose | 5 | 5 | 5 | 5 | | | | | | | | | | | | |
| D-Glucose | 5 | 5 | 5 | 5 | 5 | 5 | 5 | 5 | + | + | + | + | 4 | 5 | 5 | |
| D-Fructose | 5 | 5 | 5 | 5 | 5 | 5 | 5 | 5 | + | + | + | + | 4 | 5 | 5 | |
| D-Mannose | 3 | 5 | 5 | 5 | - | 3 | 3 | 3 | + | + | + | + | - | - | 1 | |
| L-Sorbose | | | | | | | | | | | | | | | | |
| Rhamnose | | | | | | | | | | | | | | | | |
| Dulcitol | | | | | | | | | | | | | | | | |
| Inositol | | | | | | | | | | | | | | | | |
| Mannitol | 4 | 4 | 5 | 5 | 2 | 4 | 5 | 5 | 4 | 4 | + | + | 1 | 4 | 5 | |
| Sorbitol | | | | | | | | | | | | | | | | |
| α-Methyl-D-Mannoside | | | | | | | | | | | | | | | | |
| α-Methyl-D-Glucoside | | | | | | | | | | | | | | 1 | - | - |
| N-acetyl glucosamin e | 4 | 5 | 5 | 5 | | | | | + | + | + | + | 0 | 4 | 5 | |
| Amygdaline | | | | | | | | | | | | | | | | |
| Arbutine | | | | | - | - | 1 | 2 | 1 | 3 | 4 | + | | | | |
| Esculine | 1 | 4 | 4 | 4 | 3 | 4 | 4 | 4 | 3 | 4 | + | + | 4 | 5 | 5 | |
| Salicine | 1 | 1 | 1 | 2 | - | 3 | 3 | 3 | 1 | 4 | + | + | - | - | 2 | |
| Cellobiose | | | | | | | | | | | | | | | | |
| Maltose | 3 | 4 | 5 | 5 | 2 | 4 | 5 | 5 | + | + | + | + | 1 | 3 | 3 | |
| Lactose | 5 | 5 | 5 | 5 | 5 | 5 | 5 | 5 | + | + | + | + | 2 | 5 | 5 | |
| Melbiose | | | | | | | | | - | - | 1 | 3 | - | - | 2 | |
| Saccharose | 4 | 5 | 5 | 5 | - | 1 | 2 | 1 | 3 | + | + | + | 1 | 5 | 5 | |
| Trehalose | 5 | 5 | 5 | 5 | - | 3 | 5 | 5 | 4 | + | + | + | - | 5 | 5 | |
| Inuline | | | | | | | | | | | | | | | | |
| Melezitose | | | | | | | | | | | | | | | | |
| D-Raffinose | 3 | 5 | 5 | 5 | - | 1 | 2 | 1 | - | 3 | + | + | - | 2 | 2 | |
| Amidon | | | | | | | | | | | | | | | | |
| Glycogene | | | | | | | | | | | | | | | | |
| Xylitol | | | | | | | | | | | | | | | | |
| β-Gentibiose | | | | | | | | | | | | | | | | |
| D-Turanose | | | | | | | | | | | | | | | | |
| D-Lyxose | | | | | | | | | | | | | | | | |
| D-Tagarose | | | | | | | | | | | | | | | | |
| D-Fucose | | | | | | | | | | | | | | | | |
| D-Arabitol | | | | | | | | | | | | | | | | |
| L-Arabitol | | | | | | | | | | | | | | | | |
| Gluconate | | | | | | | | | | | | | | | | |
| 2-ceto-gluconate | | | | | | | | | | | | | | | | |
| 5-ceto-gluconate | | | | | | | | | | | | | | | | |

**Table 3**

| **Metabolite formation for the strain INIX** | | | | | | | | |
|---|---|---|---|---|---|---|---|---|
| | JM 35 | | | | JM 18 | | | |
| Incubation time (h) | 24 | 48 | 72 | 96 | 24 | 48 | 72 | 96 |
| | | | | | | | | |
| Glycerol | | | | | | | | |
| Erythritol | | | | | | | | |
| D-Arabinose | | | | | | | | |
| L-Arabinose | | | | | | | | |
| Ribose | | | | | | | | |
| D-Xylose | | | | | | | | |
| L-Xylose | | | | | | | | |
| Adonitol | | | | | | | | |
| β-Methyl glycoside | | | | | | | | |
| Galactose | | | | | | | | |
| D-Glucose | 5 | 5 | 5 | 5 | 5 | 5 | 5 | 5 |
| D-Fructose | 5 | 5 | 5 | 5 | 5 | 5 | 5 | 5 |
| D-Mannose | 4 | 4 | 5 | 5 | 2 | 5 | 5 | 5 |
| L-Sorbose | | | | | | | | |
| Rhamnose | | | | | | | | |
| Dulcitol | | | | | | | | |
| Inositol | | | | | | | | |
| Mannitol | 4 | 4 | 5 | 5 | 1 | 3 | 5 | 5 |
| Sorbitol | | | | | | | | |
| α-Mathyl-D-Mannoside | | | | | | | | |
| α-Methyl-D-Glucoside | | | | | | | | |
| N-acetyl glucosamin e | 1 | 3 | 5 | 5 | - | 1 | 1 | 1 |
| Amygdaline | | | | | | | | |
| Arbutine | | | | | 1 | 1 | 2 | 2 |
| Esculine | 2 | 4 | 4 | 4 | 4 | 4 | 4 | 5 |
| Salicine | 1 | 3 | 3 | 3 | 1 | 2 | 3 | 4 |
| Cellobiose | | | | | | | | |
| Maltose | 3 | 4 | 5 | 5 | 2 | 4 | 5 | 5 |
| Lactose | 5 | 5 | 5 | 5 | 5 | 5 | 5 | 5 |
| Melbiose | | | | | | | | |
| Saccharose | 3 | 4 | 5 | 5 | - | 1 | 3 | 3 |
| Trehalose | 5 | 5 | 5 | 5 | 1- | 5 | 5 | 5 |
| Inuline | | | | | | | | |
| Melezitose | | | | | | | | |
| D-Raffinose | 1 | 3 | 5 | 5 | - | 1 | 2 | 2 |
| Amidon | | | | | | | | |
| Glycogene | | | | | | | | |
| Xylitol | | | | | | | | |
| β-Gentibiose | | | | | | | | |
| D-Turanose | | | | | | | | |
| D-Lyxose | | | | | | | | |
| D-Taqarose | | | | | | | | |
| D-Fucose | | | | | | | | |
| D-Arabitol | | | | | | | | |
| L-Arabitol | | | | | | | | |
| Gluconate | | | | | | | | |
| 2-ceto-gluconate | | | | | | | | |
| 5-ceto-gluconate | | | | | | | | |

**Table 4**

| **Metabolite fermentation for the strain K2** | | | | | | | | | | | | |
|---|---|---|---|---|---|---|---|---|---|---|---|---|
| | JM 34 | | | | JM 21 | | | | 2 JM6 | | | |
| Incubation time (h) | 24 | 48 | 72 | 96 | 24 | 48 | 72 | 96 | 24 | 48 | 72 | 96 |
| | | | | | | | | | | | | |
| Glycerol | | | | | | | | | | | | |
| Erythritol | | | | | | | | | | | | |
| D-Arabinose | | | | | | | | | | | | |
| L-Arabinose | | | | | | | | | | | | |
| Ribose | | | | | | | | | | | | |
| D-Xylose | | | | | | | | | | | | |
| L-Xylose | | | | | | | | | | | | |
| Adonitol | | | | | | | | | | | | |
| β-Methyl glycoside | | | | | | | | | | | | |
| Galactose | | | | | | | | | | | | |
| D-Glucose | 5 | 5 | 5 | 5 | 5 | 5 | 5 | 5 | + | + | + | + |
| D-Fructose | 5 | 5 | 5 | 5 | 5 | 5 | 5 | 5 | + | + | + | + |
| D-Mannose | 4 | 5 | 5 | 5 | 4 | 5 | 5 | 5 | + | + | + | + |
| L-Sorbose | | | | | | | | | | | | |
| Rhamnose | | | | | | | | | | | | |
| Dulcitol | | | | | | | | | | | | |
| Inositol | | | | | | | | | | | | |
| Mannitol | | | | | | | | | | | | |
| Sorbitol | | | | | | | | | | | | |
| α-Methyl-D-Mannoside | | | | | | | | | | | | |
| α-Methyl-D-Glucoside | | | | | | | | | | | | |
| N-acetyl glucosamine | | | | | 3 | 5 | 5 | 5 | + | + | + | + |
| Amygdaline | 1 | 1 | - | - | - | - | 3 | 5 | 1 | 4 | + | + |
| Arbutine | | | | | | | | | 1 | 4 | + | + |
| Esculine | 5 | 5 | 5 | 5 | 5 | 5 | 5 | 5 | + | + | + | + |
| Salicine | 4 | 5 | 5 | 5 | 3 | 4 | 5 | 5 | 4 | + | + | + |
| Cellobiose | 5 | 5 | 5 | 5 | 5 | 5 | 5 | 5 | + | + | + | + |
| Maltose | | | | | - | 3 | 5 | 5 | | | | |
| Lactose | 5 | 5 | 5 | 5 | 5 | 5 | 5 | 5 | 4 | + | + | + |
| Melbiose | | | | | | | | | | | | |
| Saccharose | | | | | | | | | | | | |
| Trehalose | | | | | | | | | | | | |
| Inuline | | | | | | | | | | | | |
| Melezitose | | | | | | | | | | | | |
| D-Raffinose | 1 | 0 | - | - | 2 | 4 | 5 | 5 | - | 3 | + | + |
| Amidon | | | | | | | | | | | | |
| Glycogene | | | | | | | | | | | | |
| Xylitol | | | | | | | | | | | | |
| β-Gentibiose | 4 | 5 | 5 | 5 | 4 | 4 | 4 | 4 | + | + | + | + |
| D-Turanose | | | | | | | | | | | | |
| D-Lyxose | | | | | | | | | | | | |
| D-Tagarose | | | | | | | | | | | | |
| D-Fucose | | | | | | | | | | | | |
| D-Arabitol | | | | | | | | | | | | |
| L-Arabitol | | | | | | | | | | | | |
| Gluconate | | | | | | | | | | | | |
| 2-ceto-gluconate | | | | | | | | | | | | |
| 5-ceto-gluconate | | | | | | | | | | | | |

### Strain classification to Lactobacillus kefiranofaciens species:

Comparison to 16S RNA sequences is a widely accepted means for classification of *lactobacillus* strains. The 16S gene of the five different lactobacilli strains were amplified by PCR (using forward primer SEQ ID NO:1 and reverse primer SEQ ID NO:2 sequences) and sequenced. The different strains were all phylogenically classified to the species *Lactobacillus kefiranofaciens* through an alignment of the obtained sequences with that of 80 *Lactobacillus* 16S sequences available on NCBI and of the reference strain ATCC 43761.

The corresponding complementary sequences of 16S RNA sequences of strains R2C2 (SEQ ID NO:3), INIX (SEQ ID NO:4) , BioSP (SEQ ID NO:5), K2 (SEQ ID NO:6) and ES1 (SEQ ID NO:7) are listed in the sequence listing.

### Bacterial counting procedures

The FACS method for evaluating bacterial numbers and survival was used as follows. Before performing a bacterial count using a cytometer, all aggregates have to be dissociated. Lactobacilli strains R2C2 and INIX presented herein have a tendency to form small and unstable aggregates. Dissociation can be easily achieved by resuspending the strains in PBS pyrophosphate (15 mM). Strains BioSP and K2 (particularly BioSP) form large size aggregates that cannot be dissociated using PBS pyrophosphate only. Following resuspension, these two strains must be heated at 55°C for a period of 30 minutes. Then, the strains are exposed for 15 minutes at room temperature to ethidium bromide, which binds to DNA so that each bacteria becomes detectable by cytometry. Series of dilutions (1/10, 1/100, 1/1000) are then performed to make a more precise counting.

Other methods such as plate counting method and Most Probable Number (series of inoculations with different dilutions that allow counting) can also be used.

### Growth characteristics in MRS, RCW and whey

A preculture of the different strains was prepared in RCW medium at 37°C for 12-24 hours. Cells from fresh exponentially growing cultures were counted and inoculated at a concentration of 10⁶ cfu/ml in the different media. Cultures were incubated at 37°C and cells were counted at intervals of 2, 4, 6, 8, 12, 24, 32 and 48 hours using the FACS count methods.

### Development of a direct PCR detection method

A species specific PCR amplification method was developed to allow the detection of *L*. *kefiranofaciens* in different tissues. The PCR detection test consists in an amplification of the 16S RNA gene. These primers were designed from unique *L*. *kefiranofaciens* DNA sequences identified through the results of the alignment of *Lactobacillus* 16S sequences with that of our strains and of the reference strain ATCC accession number 43761. The specificity of the primers was tested experimentally against the DNA of 5 different lactobacillus strains. This test has also been used successfully to detect *L. kefiranofaciens* DNA in experimental samples isolated from feces, colonic content, mucosa and whole colon. These results demonstrated that the primers are highly specific, detecting the presence of *L. kefiranofaciens* DNA throughout the important diversity of bacterial DNA in the intestinal flora.

*L. kefiranofaciens* specific PCR primer sequences are R2C2-16SF (SEQ ID NO:8) and R2C2-16SR (SEQ ID NO:9).

The PCR amplification cycling parameters are as follows: 94 degrees from 10 minutes followed by 30 repetitions of 94 degrees for 30 seconds, 69 degrees for 30 seconds, and 72 degrees for 1 minute, and then finishing with a single step of 72 degrees for 10 minutes. PCR products are analyzed by electrophoresis on a 2% agarose gel.

### Survival in gastric/intestinal solutions

The survival and growth of these different *Lactobacillus kefiranofaciens* strains in a low pH and gastric solutions was examined. For the experiments, the different strains were pre-cultured in RCW medium and whey at 37°C for 24 hours, before being submitted to sterile gastric solution. Survival of the different strains under acidic conditions was tested as follows.

### a) Survival of strains of lactobacillus in acid solution

Cells from fresh cultures are counted and then harvested by centrifugation, washed twice in PBS and resuspended to a concentration of 10⁷ cfu/ml in MRS broth or whey adjusted to pH 3.5, 3.0, 2.5 and 2.0. Cells are incubated at 37°C and survival measured at intervals of 15, 30, 60 and 120 min. using the FACS and plate count methods.

### b) Survival of strains of lactobacillus in human gastric solution

Cells from fresh cultures in RCW were counted and then harvested by centrifugation, washed twice in PBS and resuspended in human gastric solution to a final concentration of 10⁸ cfu/ml. After 30 minutes, 1 hour or 2 hours incubating at 37°C in human gastric solution, cultures were washed twice in PBS and resuspended in RCW broth. Survival was monitored after 24 hours of incubation at 37°C in RCW by spectrometry (640 nm) and compared with the O.D. values of non-treated cultures. The gastric solution is prepared as follows: 3.2 g/L of pepsine, 2.0 g/L of NaCl and was prepared for the purpose of this experiment at pH 2.0. Survival of the strains was excellent after 30 minutes of incubation in gastric solutions. All the strains seemed to reach the equivalent culture level as the non-treated strains as shown in Table 5.

**Table 5**

| **O.D. values for cultures incubated 30 minutes in gastric solution compared with non-treated cultures** | | |
|---|---|---|
| | | O.D. (640 nm) |
| R2C2 | non-treated | 4.63 |
| | 30 min. | 6.96 |
| INIX | non-treated | 3.29 |
| | 30 min. | 4.13 |
| BioSP | non-treated | 2.91 |
| | 30 min. | 4.94 |
| K2 | non-treated | 3.30 |
| | 30 min | 3.45 |

### Bile resistance

Cells from fresh cultures in RCW were counted and then harvested by centrifugation, washed twice in PBS and resuspended in sterile human intestinal solution to a final concentration of 10⁸ cfu/ml. After 30 minutes, 1 hour or 2 hours incubating at 37°C in intestinal solution, cultures were washed twice in PBS and resuspended in RCW broth. Survival was monitored after 24 hours of incubation at 37°C in RCW by spectrometry (640 nm) and compared with the O.D. values of non-treated cultures. The intestinal solution is prepared as follows: 10 g/L of pancreatine, 6.8 g/L of KH₂PO₄, 0.15% of bile salts and was prepared, for the purpose of this experiment, at pH 8.0. Survival of the strains was excellent after 30 minutes of incubation in intestinal solution. All the strains seemed to reach the equivalent culture level as the non-treated strains as shown in Table 6.

**Table 6**

| **O.D. values for cultures incubated 30 minutes in intestinal solution compared with non-treated cultures** | | |
|---|---|---|
| | | O.D. (640 nm) |
| R2C2 | non-treated | 4.63 |
| | 30 min. | 6.99 |
| INIX | non-treated | 3.29 |
| | 30 min. | 2.87 |
| BioSP | non-treated | 2.91 |
| | 30 min. | 3.99 |
| K2 | non-treated | 3.30 |
| | 30 min. | 3.15 |

### In vitro adhesion to CaCo-2/HT-29

The capacity of the different strains to adhere to intestinal epithelial cells was evaluated. Monolayers of CaCo-2 and HT-29 cells were allowed to grow to confluence and to differentiate for 14 days in 24-well plates. 108 cfu/ml of the different bacteria strains, previously marked with Syto9 dye (BacLight kit) were added to each well (in triplicate) in DMEM (without antibiotics) and incubated for 1 hour at 37°C. Following this, the cells were washed 4 times with PBS, the entire well content harvested by trypsin treatment for 10 minutes, and the number of bacteria per well evaluated by FACS. The strain R2C2 showed good adhesion properties on CaCo-2 cells since 15% of the bacteria were still in the well after the washes. Moreover, adhesion of R2C2 on cells was clearly higher than that of *L*. GG, a probiotic well-known for its good adhesion properties.

**Table 7**

| ***In vitro* adhesion of bacteria on human intestinal CaCo-2 cells** | |
|---|---|
| | Percentage of adherent bacteria |
| R2C2 | 15% |
| *L*. GG | 3% |

### In vitro immunomodulatory potential in co-cultures

A co-culture system of human intestinal epithelial cells (HT-29) and human PBMC is used as an *in vitro* model to evaluate the immunomodulatory effects of the bacterial strains at the intestinal level. Briefly, human HT-29 epithelial cells are seeded in the upper chamber of a transwell system (at 10⁶ cells per ml) and cultured until differentiation occurred (4 weeks in RPMI 1640 changed daily). Human peripheral blood mononuclear cells (PBMCs) are isolated by density gradient centrifugation from fresh human blood and 10⁶ cells are added to the lower chamber. The immunomodulatory effects of the different strains are evaluated by addition of 10⁶ bacteria in triplicate to either to lower or upper chamber followed by 48 hours incubation. Controls contained media alone. Following this, cell culture supernatants are removed and evaluated for extracellular cytokine levels using standard ELISA kits (R&D systems, Minneapolis, MN USA). Evaluation of cytokine expression levels is determined by RT-PCR of total RNA obtained from pooled triplicate samples of each group.

### in vitro immunomodulatory potential of whey fermented with R2C2 on human intestinal cells

A culture system of human intestinal epithelial cells (HT-29) is used as an *in vitro* model to evaluate the immunomodulatory effects of whey fermented with R2C2. Human HT-29 epithelial cells (at 10⁶ cells per ml) are cultured until differentiation occurred (4 weeks in RPMI 1640 changed daily). The immunomodulatory effect of the different strains is evaluated by addition of MPM (malleable protein matrice) in various concentrations followed by 48 hours incubation. Controls contained media alone. For determination of the anti-inflammatory potential of MPM on human intestinal cells, lipopolysaccharides (LPS) were added in the culture media to induce production of inflammatory cytokines. Following these different protocols, cell culture supernatants were removed and evaluated for extracellular cytokine levels using standard ELISA kits (R&D systems). Evaluation of cytokine expression levels was determined by RT-PCR of total RNA obtained from pooled triplicate samples of each group. Cells exposed to LPS and various concentrations of MPM showed a clear reduction of TNFα expression. Results are shown in Table 8.

**Table 8**

| ***In vitro* immunomodulation of MPM on HT-29 cells exposed to LPS** | |
|---|---|
| | Relative expression of TNFα |
| Controls | 1,0 |
| Controls+LPS | 4,2 |
| MPM 1/100+LPS | 1,3 |
| MPM 1/1000+LPS | 2,1 |
| MPM 1/10000+LPS | 2,3 |

### Example 3

### In vivo characterization of the lactobacillus strains and probiotic potential

### L. kefiranofaciens adhesion in vivo

*In vivo* adhesion and persistence of the different strains was examined. In this model, C57BU6 mice were treated by gavage (p.o.) with 10⁸ cfu/ml of the different strains for 7 days. Colon samples were then collected from 3 mice of each treatment group on day 1, day 3 and day 10 following the end of gavages to evaluate the persistence of these bacteria in the colonic tissue. The samples were opened longitudinally, colonic content collected by rinsing with saline and mucosal layer collected by scraping. Feces were also collected on the last day of gavages to evaluate the presence of the strains in treated animals. The presence of the different bacteria was evaluated by PCR, using the species specific primers developed and described above, in the feces, colonic content and mucosal samples.

### Modulation of intestinal microflora in mice

The capacity of the different strains to modulate the intestinal microflora was evaluated. C57BU6 mice were treated by gavage (p.o.) with 10⁸ cfu/ml of the different strains for 7 days. Fecal samples were then collected from each group to evaluate the levels of coliforms, lactic acid bacteria (LAB) and fecal pH was also measured. The feces are mechanically disrupted in saline and the presence of coliforms is evaluated with Perifilm Coliform Count plates™ (3M). Levels of LAB were evaluated using Petrifilm Total Aerobic Count plates, incubated anaerobically with MRS broth. All four strains tested showed a 3 to 4-fold reduction of coliforms levels in fecal samples. The strains showing the most important and constant effect are R2C2 and BioSP as shown in Table 9. Moreover, strain R2C2 showed the capacity to slightly increase LAB counts while reducing fecal pH. These effects suggest that R2C2 can possibly adhere in the intestinal tract of the animals.

**Table 9**

| **Modulation of intestinal microflora in mice** | | | |
|---|---|---|---|
| | Coliforms/mg of fece | LAB/mg of feces | Fecal pH |
| Controls | 625.24 | 9.5x10⁶ | 7.32 |
| R2C2 | 193.17 | 1.4x10⁷ | 7.18 |
| INIX | 258.64 | n/d | n/d |
| BioSP | 160.71 | n/d | n/d |
| K2 | 244.19 | n/d | n/d |

### Immunomodulatory potential Modulation of leukocytes populations

The effect of the strains to modify leukocytes cell populations was tested. C57BU6 mice were treated by gavage (p.o.) with 10⁸ cfu/ml of the different strains for 7 days. At the beginning and end of the treatment period, blood samples were collected and analyzed by flow cytometry for evaluation of leukocyte populations. The different strains showed variable effects on the leukocyte populations. The four strains seemed to modulate the mice immune system, boosting total lymphocytes and leukocytes numbers. However, only the strain R2C2 has the capacity to increase the number of polymorphonuclear (PMN) cells and only BioSP can slightly boost monocytes as shown in Table 10.

**Table 10**

| **Immunomodulation: Mice leukocyte populations following a 7-day treatment (# of cells per 20 ul of blood)** | | | | |
|---|---|---|---|---|
| | Lymphocytes | Monocytes | PMN | Total leukocytes |
| Controls | 1553.25 | 205.25 | 209.00 | 1967.50 |
| R2C2 | 2175.00 | 236.20 | 244.60 | 2655.80 |
| INIX | 2512.80 | 245.40 | 227.20 | 2985.40 |
| BioSP | 2520.75 | 281.50 | 230.75 | 3033.00 |
| K2 | 2083.75 | 194.25 | 205.50 | 2483.50 |

### L. kefiranofaciens strains in the prevention/treatment of intestinal inflammation

The potential of *L. kefiranofaciens* strains to prevent and reduce symptoms of intestinal inflammation was evaluated in the DSS-induced mouse model.

### a) Prevention of intestinal inflammation

C57BU6 mice were treated by gavage (p.o.) with 10⁸ cfu/ml of the different stains once per day for 7 days, before the induction of inflammation with DSS, and then until the end of the experiment. Intestinal inflammation is induced, on day 8, by the addition of DSS (2.5%) in the drinking water for 7 days. The level and progression of inflammation was evaluated through measurements of weight loss, diarrhea, occult blood, hematocrits, and colon lengths (post-mortem). The different treatment groups showed varying degrees of effects on the different parameters followed. Strains R2C2, BioSP and K2 showed a strong preventive effect against the development of inflammation, while strain INIX had a more moderate effect. In addition, strain L. GG showed no beneficial effect in this model, except for combined scores of occult blood and diarrhea and hematocrit. Results are shown in Tables 11-14.

Experimental groups:
Group 1: 5 mice, water + saline p.o.
Group 2: 5 mice, water-DSS + saline p.o.
Group 3: 5 mice, water-DSS + R2C2 (1x10⁸ cfu/ml) p.o.
Group 4: 5 mice, water-DSS + INIX (1x10⁸ cfu/ml) p.o.
Group 5: 5 mice, water-DSS + BioSP (1x10⁸ cfu/ml) p.o.
Group 6: 5 mice, water-DSS + K2 (1x10⁸ cfu/ml) p.o.
Group 7: 5 mice, water-DSS + *L*. GG (1x10⁸ cfu/ml) p.o.

**Table 11**

| **Weight loss (%) associated with a certain number of days consuming DSS** | | | | |
|---|---|---|---|---|
| Group | Day 4 | Day 5 | Day 6 | Day 7 |
| 2 | -0.54 | -1.87 | -5.92 | -6.50 |
| 3 | 0.10 | -0.52 | -4.00 | -4.69 |
| 4 | 0.13 | -0.88 | -4.83 | -6.41 |
| 5 | 0.44 | 0.62 | -3.39 | -2.99 |
| 6 | 2.28 | 1.95 | -2.97 | -3.46 |
| 7 | -1.10 | -1.30 | -6.09 | -6.67 |

**Table 12**

| **Hematocrit levels following 8 days of DSS exposure** | |
|---|---|
| **Group** | Hematocrit level (%) |
| 1 | 46.00 |
| 2 | 37.33 |
| 3 | 40.50 |
| 4 | 37.20 |
| 5 | 39.83 |
| 6 | 38.83 |
| 7 | 38.60 |

**Table 13**

| **Combined occult blood and diarrhea scores (evaluated on a scale of 8) following a certain number of days, consuming DSS. (More specifically the score is done as follows:** Hemoccult II (Beckman Coulter, Mississauga, Ontario, Canada) serial test slides of routine screening for fecal occult blood were used to evaluate rectal bleedings on a scale of 0-4, defined as follows : 0- No blood, 4- Feces like blood. Feces consistency was also evaluated on a scale of 0-4, defined as follows: 0-Normal consistency, 4- Liquid feces. Feces consistency and rectal bleedings values were combined and defined as the "clinical score". Scores were given by a blinded evaluator). | | |
|---|---|---|
| Group | Day 6 | Day 7 |
| 2 | 4.50 | 5.17 |
| 3 | 2.67 | 3.40 |
| 4 | 3.00 | 4.67 |
| 5 | 2.20 | 3.00 |
| 6 | 3.00 | 3.60 |
| 7 | 4.00 | 4.67 |

**Table 14**

| **Colon length (post-mortem) after 7 days consuming DSS** | |
|---|---|
| Group | Colon length (cm) |
| 1 | 7.43 |
| 2 | 5.70 |
| 3 | 6.30 |
| 4 | 5.50 |
| 5 | 6.03 |
| 6 | 5.85 |
| 7 | 5.68 |

### b) treatment of intestinal inflammation

C57BL/6 mice were treated by gavage (p.o.) with 10⁸ cfu/ml of the different strains once per day for the duration of the experiment. Intestinal inflammation was induced, on day 0, by the addition of DSS (2.5%) to the drinking water for 8 days and then replaced by fresh water for 8 days to evaluate the recovery from inflammation of the different treatment groups. The level and progression of inflammation was evaluated through measurements of weight loss, diarrhea, occult blood, hematocrits, and colon lengths (post-mortem). All *L. kefiranofaciens* strains showed positive effects, although varying in strength for the different strains, in the post-inflammatory recovery period. The strains R2C2 and BioSP showed the best potential in helping the animals recovering from DSS-induced injury. In fact, the mice receiving strain BioSP started gaining weight back 3 days before every other group. Strains R2C2 and BioSP showed better improvement in colon integrity. In addition, strain *L.* GG showed no beneficial effect in this model. These results are shown in Tables 15 and 16.

Experimental groups:
Group 1: 5 mice, water + saline p.o.
Group 2: 5 mice, water-DSS + saline p.o.
Group 3: 5 mice, water-DSS + R2C2 p.o.
Group 4: 5 mice, water-DSS + INIX p.o.
Group 5: 5 mice, water-DSS + BioSP p.o.
Group 6: 5 mice, water-DSS + K2 p.o.
Group 7: 5 mice, water-DSS + *L.* GG p.o.

**Table 15**

| **Weight variation (%) during the recovery period, after 8 days of DSS consumption** | | | | | | | | |
|---|---|---|---|---|---|---|---|---|
| Group | Day 1 | Day 2 | Day 3 | Day 4 | Day 5 | Day 6 | Day 7 | Day 8 |
| 2 | -15.37 | -16.27 | -17.09 | -16.99 | -15.75 | -14.39 | -14.48 | -13.51 |
| 3 | -17.01 | -18.79 | -18.39 | -17.56 | -14.60 | -13.29 | -13.55 | -9.75 |
| 4 | -17.13 | -17.37 | -16.90 | -14.21 | -13.06 | -11.80 | -10.83 | -7.30 |
| 5 | -14.99 | -15.72 | -13.78 | -10.36 | -8.54 | -5.77 | -6.01 | -3.58 |
| 6 | -16.95 | -16.95 | -16.41 | -16.74 | -14.16 | -13.59 | -12.21 | -9.74 |
| 7 | -20.97 | -23.21 | -25.18 | -24.69 | -22.36 | -19:98 | -17.82 | -14.24 |

**Table 16**

| **Colon length (post-mortem) after 8 days of DSS consumption and 8 days of recuperation** | |
|---|---|
| | Colon length (cm) |
| 1 | 7.44 |
| 2 | 6.13 |
| 3 | 6.55 |
| 4 | 6.32 |
| 5 | 6.70 |
| 6 | 6.42 |
| 7 | 6.15 |

### Effect of pasteurized or irradiated bacteria on intestinal inflammation

C57BL/6 mice were treated by gavage (p.o.) with 10⁸ cfu/ml of different *L. kefiranofaciens* strains once per day for the duration of the experiment. Animals received the strains R2C2 or BioSP as live, pasteurized or irradiated bacteria. Intestinal inflammation was induced by the addition of DSS (2.5%) to the drinking water for 8 days and then replaced with fresh water for 8 days to evaluate the rapidity of recovery process from inflammation for the different treatment groups. The level and progression of inflammation was evaluated through measurements of weight variation and combined scores of diarrhea and occult blood. Colon length and myeloperoxidase (MPO) activity in the colon were also evaluated at the end of the experiment. The strains R2C2 and BioSP showed positive effects, reducing weight loss during exposure to DSS, reducing combined scores of diarrhea and occult blood. Weight gain also started earlier during the post-inflammatory recovery period. Colon integrity was also better for these groups, as indicated by closer to normal length and MPO activity. No major difference was observed between groups treated with live, pasteurized or irradiated bacteria. For all these groups, a similar protective effect was observed. 5-ASA was used to compare efficacy in that experiment because of its well-known anti-inflammatory effects. For all parameters tested, 5-ASA, R2C2 and BioSP showed comparable protective effects. Results are shown in Tables 17-20.

### Experimental groups :

Group 1 : 5 mice, normal water + saline p.o.
Group 2 : 5 mice, water-DSS + saline p.o.
Group 3 : 5 mice, water-DSS + Live R2C2 p.o.
Group 4 : 5 mice, water-DSS + Pasteurized R2C2 p.o.
Group 5 : 5 mice, water-DSS + Irradiated R2C2 p.o.
Group 6 : 5 mice, water-DSS + Live BioSP p.o
Group 7 : 5 mice, water-DSS + Pasteurized BioSP p.o.
Group 8 : 5 mice, water-DSS + Irradiated BioSP p.o.
Group 9 : 5 mice, water -DSS + 5-ASA p.o

**Table 17**

| **Weight loss (%) associated with 8 days of DSS exposure** | | | | |
|---|---|---|---|---|
| Group | Day 5 | Day 6 | Day 7 | Day 8 |
| 1 | -0.20 | 2.26 | 0.35 | 1.26 |
| 2 | -1.83 | -5.23 | -8.79 | -13.5 |
| 3 | -1.59 | -3.74 | -6.03 | -8.65 |
| 4 | -1.48 | -2.23 | -5.18 | -7.02 |
| 5 | -1.83 | -3.78 | -8.47 | -10.4 |
| 6 | -1.60 | -2.69 | -5.33 | -8.51 |
| 7 | -1.80 | -3.70 | -7.04 | -9.94 |
| 8 | -2.50 | -4.73 | -7.61 | -9.18 |
| 9 | -3.21 | -3.64 | -5.90 | -8.07 |

**Table 18**

| **Weight variation (%) during a recovery period, following 8 days of DSS exposure** | | | | | | |
|---|---|---|---|---|---|---|
| Group | Day 3 | Day 4 | Day 5 | Day 6 | Day 7 | Day 8 |
| 1 | 4.23 | 3.56 | 2.29 | 1.12 | 3.26 | 5.21 |
| 2 | -25.65 | -27.36 | -29.33 | -33.27 | -32.98 | -34.0 |
| 3 | -19.05 | -18.13 | -15.03 | -14.49 | -12.63 | -11.7 |
| 4 | -18.71 | -16.31 | -11.67 | -10.72 | -9.49 | -7.22 |
| 5 | -24.57 | -27.39 | -25.57 | -23.87 | -21.71 | -20.7 |
| 6 | -23.15 | -22.54 | -20.00 | -21.06 | -17.76 | -14.1 |
| 7 | -21.33 | -21.25 | -19.61 | -21.30 | -20.01 | -13.0 |
| 8 | -18.78 | -17.05 | -15.32 | -15.80 | -14.03 | -11.1 |
| 9 | -21.64 | -20.97 | -17.66 | -17.11 | -16.96 | -11.6 |

**Table 19**

| **Combined occult blood and diarrhea scores (evaluated on a scale of 8) following a certain number of days consuming DSS** | | | | |
|---|---|---|---|---|
| Group | Day 5 | Day 6 | Day 7 | Day 8 |
| 1 | 0 | 0 | 0 | 0 |
| 2 | 2.25 | 4.50 | 6.50 | 6.50 |
| 3 | 2.00 | 1.50 | 4.75 | 5.25 |
| 4 | 2.00 | 2.80 | 2.75 | 5.25 |
| 5 | 2.00 | 2.25 | 4.00 | 5.25 |
| 6 | 1.50 | 3.75 | 4.75 | 6.00 |
| 7 | 2.00 | 3.00 | 5.20 | 5.40 |
| 8 | 1.40 | 3.00 | 4.25 | 6.00 |
| 9 | 1.75 | 3.75 | 4.25 | 5.50 |

**Table 20**

| **Colon length and MPO activity in the colon (post-mortem) following 8 days consuming DSS and 8 days of recovery** | | |
|---|---|---|
| Group | Colon length (cm) | MPO activity (U/g of tissue) |
| 1 | 7.17 | 0.056 |
| 2 | 4.79 | 0.226 |
| 3 | 5.86 | 0.081 |
| 4 | 6.06 | 0.066 |
| 5 | 5.84 | n/d |
| 6 | 6.38 | n/d |
| 7 | 6.20 | n/d |
| 8 | 6.06 | n/d |
| 9 | 5.81 | 0.043 |

### Protection against allergies

The goal of this assay is to verify the capacity of the strains to modulate an allergic response in a mouse model. BALB/c mice are immunized by i.p.(intraperitoneal) injection of OVA (ovalbumin) in Alum (Al(OH)₃ gel) on day 0 and day 14, and serum is collected on days 21, 35, and 42 to detect total IgG and OVA specific antibody response. This immunization schedule is sufficient to induce a strong allergic reaction to OVA in the control mice. The anti-allergenic effect of the *lactobacillus* strains (compared with *L*. *casei*) are evaluated in groups of mice treated orally with the different strains for the duration of the immunization protocol. Development of an allergic reaction is evaluated through the detection of total IgG production and OVA specific antibodies in the serum by ELISA. Cultured spleenocytes, from the different treatment groups are also exposed to OVA *in vitro* to evaluate the allergic reaction through cytokine and antibody production.

### Treatment of hyperlipidemia

The animal models selected to evaluate the effects of the strains on hyperlipidemia have several phenotypic parameters in common (such as hyperlipidemia, obesity and diabetes) and have been used to confirm independently the beneficial effects of the strains on these.

The different bacterial strains were tested for their capacity to regulate blood lipid levels in a rat model of hyperlipidemia. This protocol described the comparative evaluation of the different strains to niacin (vitB3), a potent hypo-lipidemic agent, in regulating artificially induced hyperlipidemia in rats. Wistar rats injected i.p. with poloxamer 407 rapidly develop severe but transient hyperlipidemia. Serum levels of glucose are also increased by this treatment. The hypo-lipidemic effect of L. *kefiranofaciens* strains is evaluated in groups of rats pre-treated orally for 7 days before injection of poloxamer. Blood lipids were measured before injection and at 24 and 72 hours following induction of hyperlipidemia. Plasma levels of triglycerides, and cholesterol (LDL) were evaluated. After a pre-treatment of 7 days, plasma triglycerides were reduced in the niacin-treated group, while a less pronounced reduction was observed in the R2C2-treated group. A reduction of plasma triglycerides was also observed in all treatment groups 72 hours after the induction of hyperlipidemia. The most important reduction was observed in the group receiving niacin (Table 21).
Group 1: 4 rats, gavage saline
Group 2: 4 rats, gavage niacin (100 mg/kg)
Group 3: 4 rats, gavage R2C2 (1x10⁸ cfu/ml)
Group 4: 4 rats, gavage INIX (1x10⁸ cfu/ml)
Group 5: 4 rats, gavage BioSP (1x10⁸ cfu/ml)
Group 6:4 rats, gavage K2 (1x10⁸ cfu/ml)

**Table 21**

| **Plasma triglycerides (mmol/L) in animals treated for 7 days and 72 hours after induction of hyperlipidemia** | | |
|---|---|---|
| Group | Triglycerides (mmol/L) after 7 days of treatment | Triglycerides (mmol/L) 72h post-induction of hyperlipidemia |
| 1 | 1.55 | 95.42 |
| 2 | 0.97 | 69.96 |
| 3 | 1.31 | 84.24 |
| 4 | 1.52 | 75.70 |
| 5 | 1.43 | 82.75 |
| 6 | 1.89 | 85.90 |

### Protection against colon cancer

The goal of the present assay was to verify the capacity of the strains to protect mice against tumor formation in the genetic model C57BL/6J-ApcMin. In this model, 100% of ApcMin heterozygous mice develop at least 30 spontaneous intestinal adenomas when exposed to a fat-rich diet. The anti-tumorigenic effect of the strains was evaluated in groups of ApcMin heterozygous mice treated orally 3 times per week with the different strains during the tumor formation period.

### Example 4

### Digestion of protein found in whey

*L. kefiranofaciens R2C2* was evaluated for its capacity to hydrolyze a protein substrate during fermentation. Whey was used as a substrate to show that the bacteria has a capacity to digest common protein found in whey such Bovine Serum Albumine (BSA), Alpha-lactalbumine (α-LAC), Beta-lactoglobilin (bLG) over time. The analysis was done by HPLC (Column RP C-4 300 A (Phenomenex, Torrance, CA, USA) with an elution gradient (Table 22).

**Table 22**

| **HPLC analysis of degradation (%) of protein** | | | |
|---|---|---|---|
| *Time (hours)* | *% of degradation α-LAC* | *% of degradation BSA* | *% of degradation bLG* |
| 0 | 100 | 100 | 100 |
| 24 | 90 | 96 | 25 |
| 48 | 81 | 93 | 4 |
| 72 | 45 | 79 | 0 |
| 96 | 24 | 64 | 0 |

### Example 5

### Anti-inflammatory potential of a whey product fermented with L. kefiranofaciens R2C2 in atopic contact dermatitis

A murine model of atopic contact dermatitis induced with oxazolone in mice was used to determine the anti-inflammatory effect of the whey fermented with *Lactobacillus kefiranofaciens* R2C2. This model of inflammation has proven to be a sensitive and useful tool to determine efficacy and potency of several anti-inflammatory and immunosuppressive drugs used in dermatological disorders like psoriasis for example. Drugs like glucocorticoids are commonly used to relieve skin and joint inflammation. Whey fermented with R2C2 administered orally either in a prophylactic (Table 24) or therapeutic fashion (Table 23) reduced the inflammation as shown with a reduction of around 30% of ear and thickness in both cases. In more details, the murine model of atopic contact dermatitis was based on those firstly described by Garrigue et al. (Contact Dermatitis., 30(4):231-273, 1994) and modified as follows: the CD-1 mice's abdomen were removed of hair and the sensitization phase was done with the application of 100 microliters of oxazolone 5 % in acetone on the abdomen (Sigma-Aldrich, Oakville, On). After 4 days, the elicitation phase (first challenge) was done with application of 50 microliters of oxazolone 5% in acetone on the right ear (25 microliters each side of the ear). The second challenge was done 7 days after the first challenge with the same procedure. The ear thickness of the mice was measured every day.

### Prophylactic protocol (MPM) - Mouse atopic contact dermatitis

The prophylactic anti-inflammatory potential of MPM (patent PCT/CA2002/01988) was evaluated firstly by the administration of MPM, 7 days prior to sensitization. Three groups of 10 CD-1 mice received by gavages, each day, 100 microliters of reconstituted lyophilized MPM, water and 1 mg of water-soluble hydrocortisone (10 mg/mL). Dermatitis was induced as described previously and ear thickness was measured every day.The therapeutic anti-inflammatory potential of MPM was evaluated by the administration of MPM only after the first challenge. Three groups of 10 CD-1 mice received by gavages, each day, 100 microliters of reconstituted lyophilized MPM, water and 1 mg of water-soluble hydrocortisone (10 mg/mL). The mouse atopic contact dermatitis was done as described previously and ear thickness was measured every day. The mice's weight was measured twice a week.

### Therapeutic protocol (R2C2) - Mouse model of atopic contact dermatitis

The therapeutic anti-inflammatory potential of R2C2 was evaluated in an animal model of atopic contact dermatitis by feeding the bacterial suspension after the first challenge. The protective effect of R2C2 was compared to that of hydrocortisone, because of its well-known anti-inflammatory effects on dermatitis. R2C2 showed a good reduction of inflammation, demonstrated by reduced ear thickness. Efficacy was comparable to that of hydrocortisone. Results are shown in Table 23-25.

**Table 23**

| **Treatment of atopic contact dermatitis (day 17)** | |
|---|---|
| **Treatment** | **Ear thickness (mm)** |
| Controls | 0,55 |
| Whey fermented with R2C2 | 0,38 |
| Hydrocortisone | 0,29 |

**Table 24**

| **Protection against atopic contact dermatitis (day 9)** | |
|---|---|
| **Treatment** | **Ear thickness (mm)** |
| Controls | 0,45 |
| Whey fermented with R2C2 | 0,32 |
| Hydrocortisone | 0,30 |

**Table 25**

| **Measurements of ear thickness (mm) during atopic contact dermatitis in laboratory animals** | | | |
|---|---|---|---|
| | Day 15 | Day 17 | Day 22 |
| Controls | 0.26 | 0.39 | 0.34 |
| R2C2 | 0.19 | 0.28 | 0.17 |
| Hydrocortisone | 0.18 | 0.27 | 0.15 |

### EXAMPLE 6.

### Anti-triglyceridemia potential of a whey product fermented with L. kefiranofaciens R2C2

### Animals

For the experiment, female Wistar rats, 7 weeks old, weighing 125-150g. were purchased from Charles River Canada. Rats were randomized into 4 different groups, each composed of at least 6 animals. Rats received a 1 mL dose of MPM, 1 mL of a saline suspension containing 10⁹ bacteria/mL of *Lactobacillus* R2C2 (the *Lactobacillus* strain used to ferment whey), 1 mL PBS (Invitrogen, Burlington, Ontario, Canada) and 100 mg/kg of niacin (Sigma) as controls. They were housed under specific pathogen-free conditions and maintained in a 24h light/dark cycle. All animals consumed standard diet and received water *ad libitum.* MPM was in a lyophilized form and prepared daily by adding 80% of water and mixed to create back a yoghurt-like product. The bacterial strain *Lactobacillus* R2C2 was routinely cultured in MRS broth (BD Biosciences, Mississauga, ON, Canada) at 37°C for a period of 24h. Bacteria were then pelleted by centrifugation at 4000 rpm for 8 minutes and re-suspended at a concentration of 10⁹ cells/mL in sterile PBS (Invitrogen). Niacin was dissolved in water in order for the rats to receive a dose of 100 mg of treatment per kg. Niacin did not solubilize perfectly so it had to be sonicated 20 minutes. The animals received the treatments for a 7-day period prior to the poloxamer 407 pluronic F-127 injection (BASF corporation, Mississauga, ON, Canada). The poloxamer 407 solution for intraperitoneal (i.p.) injection was prepared by combining the agent with sterile water and refrigerating overnight to facilitate dissolution of the polymer by the cold method of incorporation.

### Induction of hyperlipidemia

Following the 7-day treatment with the different products, all animals were made hyperlipidemic by an i.p. injection of a 300 mg dose of poloxamer 407. All syringes were placed on ice prior to poloxamer 407 administration to maintain the polymer in a mobile viscous state in order to facilitate injection, since poloxamer 407 solutions at concentrations greater than about 23 % w/w exhibit reverse thermal gelatin properties.

### Collection of blood

Approximately 1 mL of blood was collected from the jugular vein in 3 mL syringes and immediately transferred in lithium-heparinized plastic tubes (Sarstedt, Montréal, QC, Canada). Tubes were lightly shaken for 10 seconds, and then centrifuged to allow separation of the plasma. Plasma samples were collected in clean 1,5 mL eppendorf and immediately frozen at -80°C until the time of analysis. For blood collection, each animal was anesthetized using isoflurane (AErrane, Baxter Corporation, Deerfield, IL, USA). Blood collection was performed after the 7-day treatment with the different products, before poloxamer 407 injection (t=0, comparison of post-treatment lipid levels), 24 hours after the injection (t=24h, comparison of induced lipid levels) and 72h after the injection (t=72h, comparison of recovery towards normal levels). Animals were sacrificed after the last blood collection by the CO₂ asphyxia method. All procedures for the feeding of the different treatments, for the poloxamer 407 administration and subsequent blood collections were in accordance with the institution's guide for the care and use of laboratory animals and accepted by the Ethic Committee.

### Lipid analysis

Samples from all experimental groups were analyzed for total cholesterol and triglycerides. All analysis were performed in an independent laboratory (Laboratoire medical Biron, 4105-F Matte Blvd, Brossard, Québec, Canada, J4Y 2P4), ISO 9002 certified, offering a reliable plasma lipids quantification service.

R2C2 showed a slight capacity to regulate basal triglyceride levels (mmol/L) after a 7-day treatment, as shown in Table 26. The best effect was obtained with whey fermented with that bacteria. The bacteria R2C2 reduced basal triglyceride levels by close to 30% and whey fermented with R2C2 reduced basal triglyceride levels by close to 40%, similar to niacin, after 7 days of treatments. Triglyceride levels were also reduced 72h after the induction of hyperlipidemia in the R2C2-treated group, but were particularly modulated in niacin or MPM treated groups.

**Table 26**

| **Plasma triglycerides (mmol/L) in animals treated for 7 days and 72 hours after induction of hyperlipidemia** | | |
|---|---|---|
| Treatment | Triglyceride levels (7-day treatment) | Triglyceride levels (72 hours post induction of hyperlipidemia) |
| Controls | 1,83 | 95.42 |
| R2C2 | 1,30 | 84.24 |
| Whey fermented with R2C2 | 1,12 | 61.99 |
| Niacin | 1,00 | 66.96 |

**Table 27**

| **Cholesterol levels (%) in animals treated for 7 days and 72 hours after the induction of hyperlipidemia** | |
|---|---|
| Treatment | CH levels (72 hours post induction of hyperlipidemia) (%) |
| Controls | 100 |
| R2C2 | 95 |
| Whey fermented with R2C2 | 29 |
| Niacin | 33 |

### EXAMPLE 7

### Anti-hypertensive potential of a whey product fermented with L. kefiranofaciens R2C2

Anti-hypertensive potential of whey fermented with R2C2 (MPM) was evaluated by using SHR female rats (6 weeks old). 12 rats were randomized according to their weight in each treatment group. They were housed under specific pathogen-free conditions and maintained in a 12 hour- light/dark cycle. All animals consumed standard diet and received water *ad libitum.* Groups were forced-fed daily either 1 mL of water (placebo group), MPM (5 mL/kg) or Enalapril-malate (10 mg/kg), because of its well-known hypotensive effects. Systolic blood pressure (SBP) was measured weekly by the tail-cuff method with the automated RTBP2000 Tail Blood Pressure system (Kent Scientific, Torrington, CT, USA). Data collections were made weekly and an average of 3 measurements was taken as initial mean SBP. Data was acquired and analysed with Biopac Student Lab Pro® software version 3.6.1 (Biopac System, Goleta, CA, USA). After 2 weeks of treatments, Enalapril-treated group had a normalized SBP going from 184 mm Hg to 156 mm Hg. A steady decrease of the SBP was observed every week with a maximum change of at least 25 % at week 4 for the animals forced-fed with MPM. Results are shown in Table 28.

**Table 28**

| **Systolic blood pressure (mm Hg) of SHR rats receiving various treatments** | | | | | |
|---|---|---|---|---|---|
| | Week 0 | Week 1 | Week 2 | Week 3 | Week 4 |
| Controls | 185 | 181 | 184 | 183 | 187 |
| Whey fermented with R2C2 | 186 | 175 | 174 | 172 | 162 |
| Enalapril | 184 | 165 | 156 | 154 | 154 |
| Enalapril + Whey fermented with R2C2 | 185 | 155 | 150 | 145 | 148 |

### EXAMPLE 8

### Treatment of hyperlipidemia, obesity and diabetes

The animal models selected to evaluate the effects of the strains on hyperlipidemia, obesity and diabetes have several phenotypic parameters in common (such as hyperlipidemia, obesity and diabetes) and have been used to confirm independently the beneficial effects of whey fermented with L. kefiranofaciens R2C2.

### a) Model of hyperlipidemia

The whey fermented with *L*. *kefiranofaciens* R2C2 was tested for their capacity to regulate blood lipid levels in a rat model of hyperlipidemia. This protocol described the comparative evaluation to niacin (vitB3), a potent hypo-lipidemic agent, in regulating artificially induced hyperlipidemia in rats. Wistar rats injected i.p. with poloxamer 407 rapidly develop severe but transient hyperlipidemia. Serum levels of glucose are also increased by this treatment. The hypo-lipidemic effect of *L*. *kefiranofaciens* strains is evaluated in groups of rats pre-treated orally for 7 days before injection of poloxamer. Blood lipids were measured before injection and at 24 and 72 hours following induction of hyperlipidemia. Plasma levels of triglycerides, cholesterol, HDL, LDL and glucose were evaluated. After a pre-treatment of 7 days, plasma triglycerides were reduced in the niacin-treated group. A reduction of plasma triglycerides was also observed in the whey fermented with *L*. *kefiranofaciens* R2C2 group 72 hours after the induction of hyperlipidemia.
Group 1: 4 rats, gavage saline
Group 2: 4 rats, gavage niacin (100 mg/kg)
Group 3: 4 rats, gavage whey fermented with *L*. *kefiranofaciens* R2C2 (1 ml per gavage)

**Table 29**

| **Plasma triglycerides (mmol/L) in animals treated for 7 days and 72 hours after induction of hyperlipidemia** | | |
|---|---|---|
| Group | Triglycerides (mmol/L) after 7 days of treatment | Triglycerides (mmol/L) 72h post-induction of hyperlipidemia |
| 1 | 1.55 | 97.72 |
| 2 | 0.97 | 69.96 |
| 3 | 1.31 | 68.76 |

### b) Model of hypertriglyceridemia and obesity

The whey fermented with *L. kefiranofaciens* R2C2 was tested for its capacity to regulate weight gain and blood lipid levels in a diet-induced rat model of hyperlipidemia. This protocol allows a comparison to niacin (vitB3) in regulating diet-induced hyperlipidemia in rats. Wistar rats exposed to fructose (at a concentration of 10%) in their drinking water show a gradual weight gain and develop hyperlipidemia over a four week period. The hypo-lipidemic effects of the whey fermented with *L*. *kefiranofaciens* R2C2 during the fructose treatment period were evaluated. Blood samples are collected before the start of fructose treatment, and once a week for 4 weeks during the induction of hyperlipidemia. Serum levels of triglycerides were measured.
Group 1: gavage saline
Group 2: gavage niacine (100 mg/kg)
Group 3: gavage whey fermented with L. kefiranofaciens R2C2 (1 ml per gavage twice a day)
Group 4: gavage of 1 % Exopolysaccharide

**Table 30:**

| **Levels of triglyceride** | | | |
|---|---|---|---|
| Group | % relative to saline on day 0 Day 0 | % over saline group Day 7 | % over saline group Day 21 |
| 1 | 100 | 260 | 270 |
| 2 | 100 | 150 | 160 |
| 3 | 100 | 170 | 150 |
| 4 | 100 | 220 | 290 |

**Table 31**

| **Weight gain on fructose** | | | | |
|---|---|---|---|---|
| Group | % relative to day 0 Day 7 | % relative to day 0 Day 16 | % relative to day 0 Day 25 | % relative to day 0 Day 33 |
| 1 | 7 | 12 | 17 | 21 |
| 3 | 2 | 6 | 11 | 14 |
| 4 | 3 | 9 | 17 | 22 |

### c) Models of weight and fat management

The whey fermented with *L*. *kefiranofaciens* R2C2 was tested for its capacity to regulate fat distribution as well as weight gain in the Spontaneous Hypertensive Rat model and in ovariectomized rats. The SHR model was used as previously described but used to monitor the levels of visceral fat accumulating in the belly following. The animals were fed with 1 ml of whey fermented with *L*. *kefiranofaciens* R2C2 (20% solid) once a day for 56 days (P.O. q1x56)
Group 1:12 rats, gavage saline
Group 2:12 rats, gavage niacine (100 mg/kg)
Group 3:12 rats, gavage whey fermented with *L*. *kefiranofaciens* R2C2 (1 ml per gavage)

**Table 32**

| **SHR model** | |
|---|---|
| Group | % of animals with visceral fat |
| 1 | 100 |
| 2 | 70 |
| 3 | 20 |

**Table 33**

| **Ovariectomized rats** | |
|---|---|
| Forty-five 12-month-old female Wistar rats were used and randomly assigned into 2 sham-operated groups and 2 ovariectomy (OVX) groups, i.e. OVX with saline (OVX group), an OVX with whey fermented with *L*. *kefiranofaciens* R2C2 (1 ml per gavage). Daily oral administration starting on day 4 after OVX for 12 weeks. The difference is about 8% of weight gain in favor of animals fed with whey fermented with *L*. *kefiranofaciens* R2C2. | |
| Group | weight gain compared to sham-operated fed with either saline of whey fermented with *L*. *kefiranofaciens* R2C2 (1 ml per gavage) |
| 1 (OVX saline) | 1.32 X |
| 2 (OVX with whey fermented with *L*. *kefiranofaciens* R2C2 | 1.24 X |

### EXAMPLE 9

### Effect of whey fermented with L. kefiranofaciens R2C2 on human skin

Topical activity of whey fermented with *L*. kefiranofaciens R2C2 was monitored by means of sensitive and meaningful biomarkers of skin integrity such as prostaglandin E2 (PGE2) and cyclooxygenase 2 (Cox-2), both guardians of the degree of epithelial homeostasis (8). Whey fermented with *L*. *kefiranofaciens* R2C2 was compared to a nonsteroidal anti-inflammatory drug (Ibuprofen), a non selective inhibitor of Cox-2 and to an expensive commercial product with a popular brand name, Regenerist Olay®. The goal was to monitor the effect of whey fermented with L. *kefiranofaciens* R2C2 on Cox-2 expression and also basal and induced levels of PGE2 following a solar and environmental ultraviolet (UVB)-induced insult. In these experiments whey fermented with *L*. *kefiranofaciens* R2C2 was used prophylactically or therapeutically on human skin. In all experimental conditions tested, whey fermented with *L*. *kefiranofaciens* R2C2 showed a significant inhibitory effect on both biomarkers of integrity. The expression of Cox-2 was reduced following whey fermented with *L*. *kefiranofaciens* R2C2 exposure as shown by RT-PCR. Following this observation, we sought to explain the inhibition of Cox-2 by exploring the genes that could differentiate and explain this activity and rule out a potential negative affect. We found that, in addition to reducing expression of Cox-2, that the expression of 15-hydroxyprostaglandin dehydrogenase (15-PGDH), a prostaglandin-degrading enzyme that physiologically and naturally antagonizes Cox-2 was enhanced. To push further this observation, we measured the consequence of Cox-2 reduction on the biosynthesis of PGE2 in human keratinocytes and human skin exposed to whey fermented with *L*. *kefiranofaciens* R2C2. We found that whey fermented with *L*. *kefiranofaciens* R2C2 was reducing basal levels of PGE2 by about 75% after a 24-hour exposure in absence of external insult. In a situation where UVB was used as an environmental insult, whey fermented with *L*. *kefiranofaciens* R2C2 prevented the induction of PGE2 suggesting a protective role of whey fermented with *L. kefiranofaciens* R2C2. Finally, when used either before or after the UVB exposure, whey fermented with *L*. *kefiranofaciens* R2C2 exhibited the same protective activity and even a therapeutic activity as demonstrated when whey fermented with *L*. *kefiranofaciens* R2C2 was applied after the UVB exposure. It is also important to note that Regenerist Olay® was less efficacious than whey fermented with *L. kefiranofaciens* R2C2 even used non-diluted. Whey fermented with *L. kefiranofaciens* R2C2 could not be tested in a non diluted state in this experimental setting but we believe that its topical activity would even greater be increased if used non-diluted. Taken together, these data suggest that whey fermented with *L. kefiranofaciens* R2C2 exhibit an interesting biological functionality on human skin.
Effect of whey fermented with *L*. *kefiranofaciens* R2C2

### EXAMPLE 10

### Effect of whey fermented with L. kefiranofaciens R2C2

Three volunteers were willing on their own consent to consume the whey fermented with *L. kefiranofaciens* R2C2 product for various period of time. Two individuals had high cholesterol and one had diabetes and high blood pressure translating into occasional numbness in the hands. The first 2 volunteers with high cholesterol took for a period of 10 days the equivalent of 25 grams per day while the volunteer with pregnancy diabetes and high blood pressure took the product (100 ml humid) for a period of 3 weeks. The results were as follows, there was a reduction of 12% and 15% of total cholesterol for the first 2 individuals and a total alleviation of numbness and even a prevention of the pregnancy diabetes. These results are suggestive that some positive beneficial effects are to be tested in a more rigorous and rigid clinical format.

**Table 34**

| **Effect of consummation of whey fermented with *L. kefiranofaciens* R2C2 product for various period of time** | | |
|---|---|---|
| Analysis | Volunteer 1 | Volunteer 2 |
| Cholesterol total (mmol/L) | From to 4.9 to 4.2 (15% reduction) | From 7.68 to 6.77 (12%) |
| Triglycerides (mmol/L) | From 3 to 3.5 | From 1.66 to 1.33 |
| Chol LDL (Calculated) | From 2.7 to 1.9 | From 5.57 to 5.04 |
| Chols/HDL ratio | From 6.2 to 6.0 | NA |

The *Lactobacillus kefiranofaciens* of the present invention can also be used to ferment substrates like milk products, whey and cheese whey leading to beneficial product having various effects. Fermentations processes of cheese whey are used for production of a ruminant feed supplement rich in protein, in the wine production. Fermented cheese whey have also the ability to act as an antioxidant, antihypertensive, antitumor, hypolipidemic, antiviral, antibacterial, and chelating agent.

## Claims

1. A probiotic composition comprising an effective amount of *Lactobacillus kefiranofaciens* in association with a suitable carrier, wherein said *Lactobacillus kefiranofaciens* is a strain selected from the group consisting of R2C2, INIX1 K2, and ES1, for use in protecting a subject against intestinal inflammation.

2. The composition for use of claim 1, wherein an effective amount of said composition is administered.

3. The composition for use of claim 1 or 2, wherein said intestinal inflammation is caused by an inflammatory bowel disease (IBD), Crohn's disease (CD), ulcerative colitis (UC) or by an irritable bowel syndrome (IBS).

4. The composition for use of claim 1 to 3, wherein said composition is for oral, rectal or vaginal administration.

5. The composition for use of claim 1 to 4, wherein said composition is for oral administration.

6. The composition for use of claims 1 to 5, wherein said composition is administered in a form selected from the group consisting of a live bacterial population, a lyophilized bacterial population, as a fermented dairy product and as a non-viable bacterial sample.

7. The composition for use of claim 6, wherein said non-viable bacterial sample is selected from the group consisting of a heat-killed bacteria, an irradiated bacteria and a lysed bacteria.

8. The composition for use of claim 1, wherein the probiotic compound has an anti-inflammatory effect.

9. The composition for use of claims 1-8, wherein the said composition is used in association with an anti-inflammatory compound.

10. The composition for use of claim 9, wherein the anti-inflammatory compound is 5-ASA or a corticosteroid.

## Patentansprüche

1. Probiotische Zusammensetzung, umfassend eine wirksame Menge *Lactobacillus kefiranofaciens* im Zusammenhang mit einem geeigneten Träger, wobei der *Lactobacillus kefiranofaciens* ein Stamm ist, ausgewählt aus der Gruppe R2C2, INIX1 K2 und ES1, zur Verwendung zum Schutz eines Individuums gegen Darmentzündungen.

2. Zusammensetzung zur Verwendung gemäß Anspruch 1, wobei eine wirksame Menge der Zusammensetzung verabreicht wird.

3. Zusammensetzung zur Verwendung gemäß Anspruch 1 oder 2, wobei die Darmentzündung ausgelöst wird durch chronisch-entzündliche Darmerkrankungen (IBD), Morbus Crohn (CD), Colitis ulcerosa (UC) oder durch ein Reizdarmsyndrom (IBS).

4. Zusammensetzung zur Verwendung gemäß Anspruch 1 bis 3, wobei die Zusammensetzung für orale, rektale oder vaginale Verabreichung ist.

5. Zusammensetzung zur Verwendung gemäß Anspruch 1 bis 4, wobei die Zusammensetzung für orale Verabreichung ist.

6. Zusammensetzung zur Verwendung gemäß Ansprüchen 1 bis 5, wobei die Zusammensetzung in einer Form verabreicht wird, ausgewählt aus der Gruppe lebende bakterielle Population, gefriergetrocknete bakterielle Population, als ein fermentiertes Milchprodukt oder als nicht lebensfähige bakterielle Probe.

7. Zusammensetzung zur Verwendung gemäß Anspruch 6, wobei die nicht lebensfähige bakterielle Probe ausgewählt ist aus der Gruppe hitzegetötete Bakterien, bestrahlte Bakterien und lysierte Bakterien.

8. Zusammensetzung zur Verwendung gemäß Anspruch 1, wobei die probiotische Verbindung eine entzündungshemmende Wirkung hat.

9. Zusammensetzung zur Verwendung gemäß Ansprüchen 1 bis 8, wobei die Zusammensetzung im Zusammenhang mit einer entzündungshemmenden Verbindung verwendet wird.

10. Zusammensetzung zur Verwendung gemäß Anspruch 9, wobei die entzündungshemmende Verbindung 5-ASA ist oder ein Corticosteroid.

## Revendications

1. Composition probiotique comprenant une quantité effective de *Lactobacillus kefiranofaciens* associé à un porteur adéquat, où ledit *Lactobacillus kefiranofaciens* est une souche sélectionnée parmi le groupe consistant en R2C2, INIX1 K2, et ES1, pour l'utilisation dans la protection d'un sujet contre une inflammation intestinale.

2. Composition pour l'utilisation selon la revendication 1, dans laquelle une quantité effective de ladite composition est administrée.

3. Composition pour l'utilisation selon la revendication 1 ou 2, dans laquelle ladite inflammation intestinale est causée par une affection abdominale inflammatoire (IBD), la maladie de Crohn (CD), rectocolite hémorragique (UC) ou par un syndrome du côlon irritable (IBS).

4. Composition pour l'utilisation selon la revendication 1 à 3, dans laquelle ladite composition est pour une administration orale, rectale ou vaginale.

5. Composition pour l'utilisation selon la revendication 1 à 4, dans laquelle ladite composition est pour une administration orale.

6. Composition pour l'utilisation selon les revendications 1 à 5, dans laquelle ladite composition est administrée en une forme sélectionnée parmi le groupe consistant en une population bactérienne vivante, une population bactérienne lyophilisée, comme produit laitier fermenté ou comme échantillon bactérien non viable.

7. Composition pour l'utilisation selon la revendication 6, dans laquelle ledit échantillon bactérien non viable est sélectionné parmi le groupe consistant en une bactérie tuée par chaleur, une bactérie irradiée et une bactérie lysée.

8. Composition pour l'utilisation selon la revendication 1, dans laquelle le composé probiotique a un effet anti-inflammatoire.

9. Composition pour l'utilisation selon les revendications 1 à 8, dans laquelle ladite composition est utilisée en association avec un composé anti-inflammatoire.

10. Composition pour l'utilisation selon la revendication 9, dans laquelle ledit composé anti-inflammatoire est le 5-ASA ou un corticostéroïde.


### SEQUENCE LISTING

<110> Technologies Biolactis Inc.
   SIMARD, Eric
   PRECOURT, Louis-Philippe
   LEMIEUX, Pierre
<120> use of Lactobacillus kefiranofaciens as
   a probiotic and as a symbiotic.
<130> 15468-9PCT
<140> 06705161.5
   <141> 2006 02 10
<150> 60/651,657
   <151> 2005-02-11
<160> 9
<170> FastSEQ for windows version 4.0
<210> 1
   <211> 20
   <212> DNA
   <213> Artificial Sequence
<220>
   <223> sense Primer for amplifying the 16S gene of 5 different lactobacilli
<400> 1

   agagtttgat cmtggctcag 20
<210> 2
   <211> 20
   <212> DNA
   <213> Artificial Sequence
<220>
   <223> Antisense Primer for amplifying the 16S gene of 5 different lactobacilli
<400> 2
   aaggaggtga tccarccgca 20
<210> 3
   <211> 1411
   <212> DNA
   <213> Lactobacillus kefiranofacien
   <223> Strain R2C2
<400> 3
   gcagaatcac ttcggtgagg acgctgggaa agcgagcggc ggatgggtga gtaacacgtg 60
   gggaacctgc ccttaagtct gggataccac ttggaaacag gtgctaatac cggataagaa 120
   agcagttcgc atgaacagct tttaaaaggc ggcgcaagct gtcgctaaag gatggacccg 180
   cggtgcatta gctagttggt aaggtaacgg cctaccaagg cagtgatgca tagccgagtt 240
   gagagactga tcggccacat tgggactgag acacggccca aactcctacg ggaggcagca 300
   gtagggaatc ttccacaatg gacgcaagtc tgatggagca acgccgcgtg agtgaagaag 360
   gttttcggac cgtaaagctc tgttgttggt gaagaaggat agaggtagta actggccttt 420
   atttgacggt aatcaaccag aaagtcacgg ctaactacgt gccagcagcc gcggtaatac 480
   gtaggtggca agcgttgtcc ggatttattg ggcgtaaagc gagcgcaggc ggaagaataa 540
   gtctgatgtg aaagccctcg gcttaaccga ggaattgcat cggaaactgt ttttcttgag 600
   tgcagaagag gagagtagaa ctccatgtgt agcggtggaa tgcgtagata tatggaagaa 660
   taccagtggc gaagcggctc tctggtctgc aactgacgct gaggctcgaa agcatgggta 720
   gcgaacagga ttagataccc tggtagtcca tgccgtaaac gatgagtgct aagtgttggg 780
   aggcttccgc ctctcagtgc tgcagctaac gcattaagca ctccgcctgg ggagtacgac 840
   cgcaaggttg aaactcaaag gaattgacgg gggcccgcac aagcggtgga gcatgtggtt 900
   taattcgaag caacgcgaag aaccttacca ggtcttgaca tctagtgcca tttgtagaga 960
   tacaaagtcc cttcggggac gctaagacag gtggtgcatg gctgtcgtca gctcgtgtcg 1020
   tgagatgttg ggttaagtcc cgcaacgagc gcaacccttg ttattagttg ccagcattaa 1080
   gttgggcact ctaatgagac tgccggtgac aaaccggagg aaggtgggga tgacgtcaag 1140
   tcatcatgcc ccttatgacc tgggctacac acgtgctaca atgggcagca caacgagcag 1200
   cgagcctgca aaggcaagca aatctctgaa agctgttctc agttcggact gcagtctgca 1260
   actcgactgc acgaagctgg aatcgctagt aatcgcggat cagcacgccg cggtgaatac 1320
   gttcccgggc cttgtacaca ccgcccgtca caccatggga gtctgcaatg cccaaagccg 1380
   gtggcctaac cgcaaggaag gagccgtcta a 1411
<210> 4
   <211> 1411
   <212> DNA
   <213> Lactobacillus kefiranofaciens
   <223> Strain INIX
<400> 4
   gcagaatcac ttcggtgagg acgctgggaa agcgagcggc ggatgggtga gtaacacgtg 60
   gggaacctgc ccttaagtct gggataccac ttggaaacag gtgctaatac cggataagaa 120
   agcagttcgc atgaacagct tttaaaaggc ggcgcaagct gtcgctaaag gatggacccg 180
   cggtgcatta gctagttggt aaggtaacgg cctaccaagg cagtgatgca tagccgagtt 240
   gagagactga tcggccacat tgggactgag acacggccca aactcctacg ggaggcagca 300
   gtagggaatc ttccacaatg gacgcaagtc tgatggagca acgccgcgtg agtgaagaag 360
   gttttcggac cgtaaagctc tgttgttggt gaagaaggat agaggtagta actggccttt 420
   atttgacggt aatcaaccag aaagtcacgg ctaactacgt gccagcagcc gcggtaatac 480
   gtaggtggca agcgttgtcc ggatttattg ggcgtaaagc gagcgcaggc ggaagaataa 540
   gtctgatgtg aaagccctcg gcttaaccga ggaattgcat cggaaactgt ttttcttgag 600
   tgcagaagag gagagtagaa ctccatgtgt agcggtggaa tgcgtagata tatggaagaa 660
   taccagtggc gaagcggctc tctggtctgc aactgacgct gaggctcgaa agcatgggta 720
   gcgaacagga ttagataccc tggtagtcca tgccgtaaac gatgagtgct aagtgttggg 780
   aggcttccgc ctctcagtgc tgcagctaac gcattaagca ctccgcctgg ggagtacgac 840
   cgcaaggttg aaactcaaag gaattgacgg gggcccgcac aagcggtgga gcatgtggtt 900
   taattcgaag caacgcgaag aaccttacca ggtcttgaca tctagtgcca tttgtagaga 960
   tacaaagtcc cttcggggac gctaagacag gtggtgcatg gctgtcgtca gctcgtgtcg 1020
   tgagatgttg ggttaagtcc cgcaacgagc gcaacccttg ttattagttg ccagcattaa 1080
   gttgggcact ctaatgagac tgccggtgac aaaccggagg aaggtgggga tgacgtcaag 1140
   tcatcatgcc ccttatgacc tgggctacac acgtgctaca atgggcagca caacgagcag 1200
   cgagcctgca aaggcaagca aatctctgaa agctgttctc agttcggact gcagtctgca 1260
   actcgactgc acgaagctgg aatcgctagt aatcgcggat cagcacgccg cggtgaatac 1320
   gttcccgggc cttgtacaca ccgcccgtca caccatggga gtctgcaatg cccaaagccg 1380
   gtggcctaac cgcaaggaag gagccgtcta a 1411
<210> 5
   <211> 1411
   <212> DNA
   <213> Lactobacillus kefiranofaciens
   <223> Strain BioSP
<400> 5
   gcagaatcac ttcggtgagg acgctgggaa agcgagcggc ggatgggtga gtaacacgtg 60
   gggaacctgc ccttaagtct gggataccac ttggaaacag gtgctaatac cggataagaa 120
   agcagttcgc atgaacagct tttaaaaggc ggcgcaagct gtcgctaaag gatggacccg 180
   cggtgcatta gctagttggt aaggtaacgg cctaccaagg cagtgatgca tagccgagtt 240
   gagagactga tcggccacat tgggactgag acacggccca aactcctacg ggaggcagca 300
   gtagggaatc ttccacaatg gacgcaagtc tgatggagca acgccgcgtg agtgaagaag 360
   gttttcggac cgtaaagctc tgttgttggt gaagaaggat agaggtagta actggccttt 420
   atttgacggt aatcaaccag aaagtcacgg ctaactacgt gccagcagcc gcggtaatac 480
   gtaggtggca agcgttgtcc ggatttattg ggcgtaaagc gagcgcaggc ggaagaataa 540
   gtctgatgtg aaagccctcg gcttaaccga ggaattgcat cggaaactgt ttttcttgag 600
   tgcagaagag gagagtagaa ctccatgtgt agcggtggaa tgcgtagata tatggaagaa 660
   taccagtggc gaagcggctc tctggtctgc aactgacgct gaggctcgaa agcatgggta 720
   gcgaacagga ttagataccc tggtagtcca tgccgtaaac gatgagtgct aagtgttggg 780
   aggcttccgc ctctcagtgc tgcagctaac gcattaagca ctccgcctgg ggagtacgac 840
   cgcaaggttg aaactcaaag gaattgacgg gggcccgcac aagcggtgga gcatgtggtt 900
   taattcgaag caacgcgaag aaccttacca ggtcttgaca tctagtgcca tttgtagaga 960
   tacaaagtcc cttcggggac gctaagacag gtggtgcatg gctgtcgtca gctcgtgtcg 1020
   tgagatgttg ggttaagtcc cgcaacgagc gcaacccttg ttattagttg ccagcattaa 1080
   gttgggcact ctaatgagac tgccggtgac aaaccggagg aaggtgggga tgacgtcaag 1140
   tcatcatgcc ccttatgacc tgggctacac acgtgctaca atgggcagca caacgagcag 1200
   cgagcctgca aaggcaagca aatctctgaa agctgttctc agttcggact gcagtctgca 1260
   actcgactgc acgaagctgg aatcgctagt aatcgcggat cagcacgccg cggtgaatac 1320
   gttcccgggc cttgtacaca ccgcccgtca caccatggga gtctgcaatg cccaaagccg 1380
   gtggcctaac cgcaaggaag gagccgtcta a                               1411
<210> 6
   <211> 1411
   <212> DNA
   <213> Lactobacillus kefiranofaciens
   <223> Strain K2
<400> 6
   gcagaatcac ttcggtgagg acgctgggaa agcgagcggc ggatgggtga gtaacacgtg 60
   gggaacctgc ccttaagtct gggataccac ttggaaacag gtgctaatac cggataagaa 120
   agcagttcgc atgaacagct tttaaaaggc ggcgcaagct gtcgctaaag gatggacccg 180
   cggtgcatta gctagttggt aaggtaacgg cctaccaagg cagtgatgca tagccgagtt 240
   gagagactga tcggccacat tgggactgag acacggccca aactcctacg ggaggcagca 300
   gtagggaatc ttccacaatg gacgcaagtc tgatggagca acgccgcgtg agtgaagaag 360
   gttttcggac cgtaaagctc tgttgttggt gaagaaggat agaggtagta actggccttt 420
   atttgacggt aatcaaccag aaagtcacgg ctaactacgt gccagcagcc gcggtaatac 480
   gtaggtggca agcgttgtcc ggatttattg ggcgtaaagc gagcgcaggc ggaagaataa 540
   gtctgatgtg aaagccctcg gcttaaccga ggaattgcat cggaaactgt ttttcttgag 600
   tgcagaagag gagagtagaa ctccatgtgt agcggtggaa tgcgtagata tatggaagaa 660
   taccagtggc gaagcggctc tctggtctgc aactgacgct gaggctcgaa agcatgggta 720
   gcgaacagga ttagataccc tggtagtcca tgccgtaaac gatgagtgct aagtgttggg 780
   aggcttccgc ctctcagtgc tgcagctaac gcattaagca ctccgcctgg ggagtacgac 840
   cgcaaggttg aaactcaaag gaattgacgg gggcccgcac aagcggtgga gcatgtggtt 900
   taattcgaag caacgcgaag aaccttacca ggtcttgaca tctagtgcca tttgtagaga 960
   tacaaagttc cttcggggac gctaagacag gtggtgcatg gctgtcgtca gctcgtgtcg 1020
   tgagatgttg ggttaagtcc cgcaacgagc gcaacccttg ttattagttg ccagcattaa 1080
   gttgggcact ctaatgagac tgccggtgac aaaccggagg aaggtgggga tgacgtcaag 1140
   tcatcatgcc ccttatgacc tgggctacac acgtgctaca atgggcagca caacgagcag 1200
   cgagcctgca aaggcaagca aatctctgaa agctgttctc agttcggact gcagtctgca 1260
   actcgactgc acgaagctgg aatcgctagt aatcgcggat cagcacgccg cggtgaatac 1320
   gttcccgggc cttgtacaca ccgcccgtca caccatggga gtctgcaatg cccaaagccg 1380
   gtggcctaac cgcaaggaag gagccgtcta a                               1411
<210> 7
   <211> 1384
   <212> DNA
   <213> Lactobacillus kefiranofaciens
   <223> Strain ES1
<400> 7
   agaatcactt cggtgaggac gctgggaaag cgagcggcgg atgggtgagt aacacgtggg 60
   gaacctgccc ttaagtctgg gataccactt ggaaacaggt gctaataccg gataagaaag 120
   cagttcgcat gaacagcttt taaaaggcgg cgcaagctgt cgctaaagga tggacccgcg 180
   gtgcattagc tagttggtaa ggtaacggcc taccaaggca gtgatgcata gccgagttga 240
   gagactgatc ggccacattg ggactgagac acggcccaaa ctcctacggg aggcagcagt 300
   agggaatctt ccacaatgga cgcaagtctg atggagcaac gccgcgtgag tgaagaaggt 360
   tttcggaccg taaagctctg ttgttggtga agaaggatag aggtagtaac tggcctttat 420
   ttgacggtaa tcaaccagaa agtcacggct aactacgtgc cagcagccgc ggtaatacgt 480
   aggtggcaag cgttgtccgg atttattggg cgtaaagcga gcgcaggcgg aagaataagt 540
   ctgatgtgaa agccctcggc ttaaccgagg aattgcatcg gaaactgttt ttcttgagtg 600
   cagaagagga gagtagaact ccatgtgtag cggtggaatg cgtagatata tggaagaata 660
   ccagtggcga agcggctctc tggtctgcaa ctgacgctga ggctcgaaag catgggtagc 720
   gaacaggatt agataccctg gtagtccatg ccgtaaacga tgagtgctaa gtgttgggag 780
   gcttccgcct ctcagtgctg cagctaacgc attaagcact ccgcctgggg agtacgaccg 840
   caaggttgaa actcaaagga attgacgggg gcccgcacaa gcggtggagc atgtggttta 900
   attcgaagca acgcgaagaa ccttaccagg tcttgacatc tagtgccatt tgtagagata 960
   caaagttcct tcggggacgc taagacaggt ggtgcatggc tgtcgtcagc tcgtgtcgtg 1020
   agatgttggg ttaagtcccg caacgagcgc aacccttgtt attagttgcc agcattaagt 1080
   tgggcactct aatgagactg ccggtgacaa accggaggaa ggtggggatg acgtcaagtc 1140
   atcatgcccc ttatgacctg ggctacacac gtgctacaat gggcagcaca acgagcagcg 1200
   agcctgcgaa ggcaagcaaa tctctgaaag ctgttctcag ttcggactgc agtctgcaac 1260
   tcgactgcac gaagctggaa tcgctagtaa tcgcggatca gcacgccgcg gtgaatacgt 1320
   tcccgggcct tgtacacacc gcccgtcaca ccatgggagt ctgcaatgcc caaagccggt 1380
   ggcc                                                              1384
<210> 8
   <211> 24
   <212> DNA
   <213> Artificial sequence
<220>
   <223> sense PCR primer for specifically amplifying the 16S gene of L. kefiranofaciens R2C2
<400> 8
   taagaaagca gttcgcatga acag 24
<210> 9
   <211> 24
   <212> DNA
   <213> Artificial sequence
<220>
   <223> Antisense PCR primer for specifically amplifying the 16S gene of L. kefiranofaciens
   R2C2
<400> 9
   gggactttgt atctctacaa atgg 24